# EUROPÄISCHE PATENTSCHRIFT

(11) **EP 1 942 988 B1**
(45) Veröffentlichungstag und Bekanntmachung des Hinweises auf die Patenterteilung: **22.08.2012**
(21) Anmeldenummer: 06805977.3
(22) Anmeldetag: 30.09.2006
(51) Int. Cl.: A61Q 5/06, A61K 8/02, A61K 8/25

(54) **PULVERFÖRMIGES STYLINGMITTEL**
PULVERULENT STYLING COMPOSITION
AGENTS COIFFANTS PULVERULENTS

(30) Priorität: 02.11.2005 DE 102005052585
(43) Veröffentlichungstag der Anmeldung: 16.07.2008
(73) Patentinhaber: Henkel AG & Co. KGaA, 40589 Düsseldorf (DE)
(72) Erfinder: HENTRICH, Dirk, 22457 Hamburg (DE); RICHTERS, Bernd, 21129 Hamburg (DE)
(86) Internationale Anmeldenummer: PCT/EP2006/009515
(87) Internationale Veröffentlichungsnummer: WO 2007/051511

(56) Entgegenhaltungen:
- EP-A2- 1 426 033
- WO-A-01/37800
- WO-A-03/037287
- WO-A-2005/058256
- HASENZAHL S ET AL: "Fumed silica for personal care and cosmetics - versatile and effective" SOFW-JOURNAL SEIFEN, OELE, FETTE, WACHSE, VERLAG FUR CHEMISCHE INDUSTRIE, AUGSBURG, DE, August 2003 (2003-08), Seiten 1-8, XP002289365 ISSN: 0942-7694 in der Anmeldung erwähnt
- "Cosmetic powder obtd. using hydrophobic silicate - water and lubricant e.g. nylon or polyethylene powder, provides a cooling feeling" DERWENT, 1983, XP002276355

## Beschreibung

Die vorliegende Erfindung betrifft die Verwendung einer pulverförmigen Zusammensetzung auf Basis eines hydrophobierten Siliciumdioxidpulvers als Stylingmittel zur temporären Verformung keratinischer Fasern und ein entsprechendes pulverförmiges Stylingmittel.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Die bekannten Formen temporärer Stylingmittel erfordern zudem in der Regel eine große Menge an Hilfsstoffen, die nicht der eigentlichen Gestaltung der Frisur, sondern der Formulierung des jeweiligen Mittels dienen. So enthalten die Stylingmittel oftmals große Mengen organischer Lösungsmittel. Die Konfektionierung als Haarspray erfordert zudem weitere organische Verbindungen, die als Treibmittel Verwendung finden. Dies hat zum einen die Folge, dass die Umwelt mit flüchtigen organischen Verbindungen (VOC) belastet wird, zum anderen erhöht sich so das Produktvolumen und damit das Volumen der benötigten Verpackungen erheblich.

Aufgabe der vorliegenden Erfindung war es daher, ein Haarbehandlungsmittel zur temporären Formgebung zur Verfügung zur stellen, das einerseits ein hervorragendes und haltbares Stylingergebnis ergibt, und andererseits in möglichst kompakter Form vorliegt und genau und einfach dosiert werden kann.

Es wurde nunmehr gefunden, dass dies auf einfache Weise durch ein Stylingmittel erreicht werden kann, das in Pulverform vorliegt.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa Siliciumdioxid verwendet werden. Besonders interessant ist hydrophobiertes Siliciumdioxid. Dieses kann beispielsweise ausgehend von pyrogenem Siliciumdioxid, das in verschiedenen Spezifikationen kommerziell erhältlich ist, erhalten werden. Auf der Oberfläche trägt unbehandeltes pyrogenes Siliciumdioxid Silanol- und Siloxangruppen. Dadurch hat es eine hohe Affinität zu Wasser, d.h. es ist hydrophil. Durch Umsetzung mit geeigneten organischen Siliciumverbindungen lassen sich Alkylsilylgruppen auf der Oberfläche des pyrogenen Siliciumdioxids chemisch binden. Es entstehen modifizierte Siliciumdioxidpulver, die nicht mehr von Wasser benetzt werden, d.h. die hydrophobe Eigenschaften aufweisen.

In Seifen, Öle, Fette, Wachse (SOFW), 129(8) (2003), S. 22-32 wird der Einsatz von hydrophobiertem Siliciumdioxid in der Kosmetik zur Herstellung von so genanntem trockenen Wasser für die Haut beschrieben. Dabei werden die hydrophoben Eigenschaften des modifizierten Siliciumdioxids ausgenutzt, die bewirken, dass das Siliciumdioxid beim intensiven Mischen mit Wasser nicht einfach in diesem dispergiert wird. Die Wassertropfen werden vielmehr von den hydrophoben Feststoffpartikeln umhüllt und am erneuten Zusammenfließen gehindert. Auf diese Weise lassen sich pulverförmige Feststoffe mit einem Wassergehalt von bis zu über 95 % erhalten. Unter mechanischer Beanspruchung, beispielsweise beim Verreiben auf der Haut, wird das eingeschlossene Wasser wieder freigesetzt. Dieses trockene Wasser wird als Grundlage zur Herstellung von lagerstabilem, festem Wasserstoffperoxid und von verstreichbaren Zubereitungen mit sehr geringem Ölgehalt beschrieben.

Dieses Konzept liegt auch der in EP 1 235 554 B1 beschriebenen Herstellung kosmetischer oder pharmazeutischer, verflüssigbarer Pulverzusammensetzungen zu Grunde. Die Pulverzusammensetzungen umfassen hydrophob beschichtete Siliciumdioxidteilchen, in die Wasser und ein wasserlösliches Polymer eingeschlossen sind, wobei die Zusammensetzungen weniger als 1 % Öl enthalten. Durch Zugabe des wasserlöslichen Polymers soll erreicht werden, dass sich das Pulver bei der Anwendung auf der Haut angenehm und nicht körnig anfühlt, ohne dass zu diesem Zweck dem Produkt eine Ölkomponente zugegeben werden müsste. Das Polymer wird zu diesem Zweck der Wasserphase in einer Menge von 0,01 bis 5 Gew.-% zugegeben, wobei ein Gehalt an lediglich 0,1 bis 1 Gew.-% bevorzugt ist. Die verflüssigbaren Pulverzusammensetzungen werden vor allem zur Herstellung dekorativer Kosmetika eingesetzt. Daneben sind auch der Einsatz in Deodorants oder Sonnenschutzmitteln, oder die Anwendung auf dem Haar als Basis von Haarbehandlungsmitteln, die Perlglanzmittel oder Pflegekomponenten enthalten, beschrieben. Eine Verwendung im Bereich der Stylingmittel ist nicht genannt.

WO 03/037287 A1 offenbart die Verwendung eines Granulats auf Basis von pyrogenem Siliciumdioxid in kosmetischen Zusammensetzungen. Die speziellen Granulate können silanisiert, d.h. hydrophobiert werden und eignen sich zur Herstellung kosmetischer Zusammensetzungen jeglicher Konsistenz, beispielsweise Flüssigkeiten, Schäume, Sprays oder Pulver. Als mögliche kosmetische Zusammensetzungen werden eine Vielzahl denkbarer Kosmetika, unter anderem Haarstylingmittel, genannt. Dabei werden jedoch nur die üblichen Applikationsformen Lotion, Haarspray, Haarlack, Haargel und Haarwachs genannt. Ein Hinweis darauf, dass auf Basis des beschriebenen Siliciumdioxids pulverförmige Stylingmittel hergestellt werden könnten, findet sich nicht.

Ein erster Gegenstand der vorliegenden Erfindung ist daher die Verwendung einer pulverförmigen Zusammensetzung, enthaltend 50 bis 95 Gew.-% eines wässrigen Lösungsmittels, hydrophobiertes Siliciumdioxidpulver und mindestens ein filmbildendes und/oder festigendes Polymer zur temporären Verformung keratinischer Fasern.

Unter keratinischen Fasern sind dabei erfindungsgemäß Pelze, Wolle, Federn und insbesondere menschliche Haare zu verstehen.

Die pulverförmigen Zusammensetzungen können sehr einfach dosiert werden. Sie lassen sich zudem sehr gleichmäßig im Haar verteilen, da das Lösungsmittel und das filmbildende und/oder festigende Polymer erst unter mechanischer Beanspruchung freigesetzt werden. Das Pulver kann also zunächst vorsichtig im Haar verteilt und erst anschließend stärker mechanisch belastet werden, beispielsweise durch gezieltes Einmassieren des Pulvers in das Haar. Dadurch wird das haltgebende Polymer erst direkt auf der gewünschten Haarpartie freigesetzt. So lässt sich sehr gezielt eine hervorragende Stylingwirkung erreichen.

Vorzugsweise wird eine pulverförmige Zusammensetzung verwendet, die 70 bis 90 Gew.-%, besonders bevorzugt 80 bis 90 Gew.-% eines wässrigen Lösungsmittels, bezogen auf die gesamte Zusammensetzung, enthält.

Unter einem wässrigen Lösungsmittel wird dabei Wasser oder ein Gemisch aus Wasser und einem C₁-C₄-Alkohol, insbesondere Ethanol, verstanden. Da oberflächenaktive Substanzen und Alkohole unter Umständen jedoch hydrophobiertes Siliciumdioxid benetzen und damit die hydrophoben Eigenschaften negativ beeinflussen können, kann es je nach Art des eingesetzten hydrophobierten Siliciumdioxids notwendig sein, den Gehalt an C₁-C₄-Alkohol im wässrigen Lösungsmittel unter einer kritischen Maximalmenge zu halten.

Vorzugsweise wird daher als wässriges Lösungsmittel Wasser oder ein Gemisch aus Wasser und maximal 60 Gew.-% C₁-C₄-Alkohol, bezogen auf das Lösungsmittelgemisch, eingesetzt. Besonders bevorzugte wässrige Lösungsmittel sind Wasser oder ein Gemisch aus Wasser und maximal 30 Gew.-% C₁-C₄-Alkohol, bezogen auf das Lösungsmittelgemisch. Ganz besonders bevorzugt wird Wasser eingesetzt.

Die verwendeten pulverförmigen Zusammensetzungen enthalten hydrophobiertes Siliciumdioxid. Die Art des hydrophobierten Siliciumdioxids ist nicht prinzipiell beschränkt, solange sichergestellt ist, dass beim intensiven Vermischen mit dem wässrigen Lösungsmittel, enthaltend mindestens ein filmbildendes und/oder festigendes Polymer und gegebenenfalls weitere Inhaltsstoffe, ein pulverförmiges Produkt entsteht.

Geeignete hydrophobierte Siliciumdioxide sind bekannt und beispielsweise in Seifen, Öle, Fette, Wachse (SÖFW), 3 (2004), S. 4-13, EP 1 235 554 B1, WO 03/037287 A1 und EP 0 725 037 B1 beschrieben.

Vorzugsweise werden hydrophobierte Siliciumdioxide eingesetzt, die durch Silanisierung von pyrogenem Siliciumdioxid erhalten werden.

Bevorzugt sind solche hydrophobierte Siliciumdioxide, die eine spezifische Oberfläche nach BET zwischen 10 und 400, vorzugsweise zwischen 80 bis 300 m²/g aufweisen.

Eine Vielzahl geeigneter hydrophobierter Siliciumdioxide ist kommerziell erhältlich. Beispielhaft seien Aerosil^{®} R104 V, Aerosil^{®} R106, Aerosil^{®} R202, Aerosil^{®} R805, Aerosil^{®} R812, Aerosil^{®} R812S, Aerosil^{®} R972 und Aerosil^{®} R8200, alle Degussa, sowie HDK^{®} H2000, HDK^{®} H2050 und HDK^{®} H3004, alle Wacker, genannt.

Besonders bevorzugt werden die hydrophobierten Siliciumdioxide eingesetzt, die unter den Bezeichnungen Aerosil^{®} R202, Aerosil^{®} R812S oder Aerosil^{®} R972 erhältlich sind. Ganz besonders bevorzugt kommt das Siliciumdioxid mit der INCl-Bezeichnung Silica Silylate zum Einsatz, das von der Firma Degussa unter der Bezeichnung Aerosil^{®} R812S vertrieben wird.

Die eingesetzten pulverförmigen Zusammensetzungen enthalten das hydrophobierte Siliciumdioxidpulver vorzugsweise in einer Menge von 0,5 bis 15 Gew.%, bezogen auf die gesamte pulverförmige Zusammensetzung. Die optimale Menge hängt dabei vor allem von der Hydrophobizität des eingesetzten Siliciumdioxidpulvers ab. Je hydrophober das Siliciumdioxidpulver ist, desto weniger davon wird benötigt, um ein stabiles, pulverförmiges Produkt zu erhalten. Das vergleichsweise hydrophobe Siliciumdioxid, das von der Firma Degussa unter der Bezeichnung Aerosil^{®} R812S vertrieben wird, wird beispielsweise besonders bevorzugt in einer Menge von 3 bis 8 Gew.-%, bezogen auf die gesamte pulverförmige Zusammensetzung, eingesetzt, das weniger hydrophobe Siliciumdioxid Aerosil^{®} R972 in einer Menge von 10 bis 15 Gew.-%, bezogen auf die gesamte pulverförmige Zusammensetzung.

Die verwendete pulverförmige Zusammensetzung enthält als weitere zwingende Komponente mindestens ein filmbildendes und/oder festigendes Polymer.

Das filmbildende und/oder festigende Polymer ist in der pulverförmigen Zusammensetzung vorzugsweise in einer Menge von 1 bis 15 Gewichtsprozent, besonders bevorzugt von 5,5 bis 15 Gewichtsprozent, ganz besonders bevorzugt in einer Menge von 6 bis 10 Gewichtsprozent, bezogen auf die gesamte pulverförmige Zusammensetzung, enthalten. Selbstverständlich können auch mehrere filmbildende und/oder festigende Polymere enthalten sein. Dabei können diese filmbildenden und/oder festigenden Polymere sowohl permanent als auch temporär kationisch, anionisch, nichtionisch oder amphoter sein. Bei der Verwendung von mindestens zwei filmbildenden und/oder festigenden Polymeren können diese selbstverständlich unterschiedliche Ladungen aufweisen. Erfindungsgemäß bevorzugt kann es sein, wenn ein ionisches filmbildendes und/oder festigendes Polymer mit einem amphoteren und/oder nichtionischen filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Viele Polymere, die primär als filmbildend beschrieben werden, haben auch festigende Eigenschaften und umgekehrt. Es wird an dieser Stelle daher explizit darauf verwiesen, dass im Rahmen der vorliegenden Erfindung sowohl filmbildende als auch festigende Polymere wesentlich sind. Da beide Eigenschaften nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Wasser, Alkohol oder Wasser/Alkohol-Gemischen besitzen. So lassen sich entsprechende Lösungen herstellen, die sich auf einfache Art und Weise anwenden bzw. weiterverarbeiten lassen. Die filmbildenden Polymere können synthetischen oder natürlichen Ursprungs sein.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete und erfindungsgemäß bevorzugt eingesetzte synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁- bis C₇-Alkylgruppen, besonders bevorzugt C₁-bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol-Copolymere, die beispielsweise, unter den Handelsbezeichnungen Ucon^{®} der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI^{®} von der Firma Lehmann & Voss, Hamburg, vertrieben wird.

Festigende Polymere tragen zum Halt und/oder zum Aufbau des Haarvolumens und der Haarfülle der Gesamtfrisur bei. Diese sogenannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein langanhaltender Frisurenaufbau und -erhalt gewährleistet. Als Testmethode hierfür wird häufig der sogenannte curl-retention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren nach der jeweiligen Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, AcrylatesNA Copolymer, AcrylatesNP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl StearateNA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquatemium-7, Polyquaternium-8, Polyquaternium-9, Polyquaternium-10, Polyquaternium-11, Polyquaternium-12, Polyquaternium-13, Polyquaternium-14, Polyquaternium-15, Polyquatemium-16, Polyquaternium-17, Polyquaternium-18, Polyquaternium-19, Polyquaternium-20, Polyquaternium-22, Polyquatemium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquatemium-37, Polyquaternium-39, Polyquaternium-45, Polyquaternium-46, Polyquaternium-47, Polyquaternium-48, Polyquaternium-49, Polyquatemium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, VinylamineNinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VPNA Copolymer, VPNinyl Caprolactam/DMAPA Acrylates Copolymer, Yeast Palmitate.

Vorzugsweise werden erfindungsgemäß pulverförmige Zusammensetzungen verwendet, die mindestens ein filmbildendes und/oder festigendes Polymer enthalten, das aus Vinylpyrrolidon-Vinylacetat-Copolymeren, Vinylacetat-Crotonsäure-Copolymeren, Vinylcaprolactam-Vinylpyrrolidon-Dimethylaminoethylmethacrylat-Copolymeren, Octylacrylamid-Acrylat-Butylaminoethyl-Methacrylat-Copolymeren und quaternierten Vinylpyrrolidon-Dimethylaminoethylmethacrylat-Copolymeren ausgewählt ist.

Besonders bevorzugt handelt es sich bei dem filmbildenden und/oder festigenden Polymer um die Vinylpyrrolidon-Vinylacetat-Copolymeren Luviskol^{®} VA 37 oder PVPNA Copolymer 60/40 W NP, das Vinylacetat-Crotonsäure-Copolymere, das unter der Handelsbezeichnung Aristoflex^{®} A 60 vertrieben wird, das Vinylcaprolactam-Vinylpyrrolidon-Dimethylaminoethylmethacrylat-Copolymer mit der Handelsbezeichnung Advantage^{®} LC-E, das unter der Bezeichnung Amphomer^{®} erhältliche amphotere Octylacrylamid-Acrylat-Butylaminoethyl-Methacrylat-Copolymer oder das durch Umsetzung mit Diethylsulfat quaternierte Vinylpyrrolidon-Dimethylaminoethylmethacrylat-Copolymer, das unter der Handelsbezeichnung Gafquat^{®} 755N vertrieben wird.

Insbesondere bevorzugt werden pulverförmige Zusammensetzungen eingesetzt, die wenigstens ein Vinylpyrrolidon-Vinylacetat-Copolymer enthalten.

Die verwendete pulverförmige Zusammensetzung kann weiterhin die Hilfs- und Zusatzstoffe enthalten, die üblicherweise herkömmlichen Stylingmitteln zugesetzt werden.

Als geeignete Hilfs- und Zusatzstoffe sind insbesondere Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken dabei die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Silicium-organischer Verbindungen. Hierunter werden zunächst Dimethiconole und Dimethicone, etwa das von der Firma Dow Corning unter der Bezeichnung Dow Corning ^{®} 193 Surfactant vertriebene PEG-12 Dimethicone, verstanden. Diese können sowohl linear als auch verzweigt als auch cyclisch oder cyclisch und verzweigt sein. Weiterhin fallen darunter Dimethiconcopolyole, wie sie beispielsweise von der Firma Dow Corning unter der Bezeichnung Dow Corning ^{®} 5330 Fluid vertrieben werden, und aminofunktionelle Silikone, insbesondere die Silikone, die unter der INCI-Bezeichnung Amodimethicone zusammengefasst sind.

Als Pflegestoff kann auch ein kationisches Tensid eingesetzt werden. Bevorzugt sind dabei kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Da sich der Zusatz oberflächenaktiver Substanzen jedoch negativ auf die hydrophoben Eigenschaften des hydrophobierten Siliciumdioxids und damit auf die Stabilität der verwendeten pulverförmigen Zusammensetzungen auswirken kann, ist die Menge an pflegendem Tensid sorgfältig auf die Gesamtzusammensetzung abzustimmen. Vorzugsweise wird auf den Zusatz tensidischer Bestandteile verzichtet.

Als Pflegestoff eignen sich ebenfalls pflegende Polymere.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Ein besonders geeignetes Homopolymer ist das, gewünschtenfalls vernetzte, Poly(methacryloyloxyethyltrimethylammoniumchlorid) mit der INCI-Bezeichnung Polyquatemium-37. Die Vernetzung kann. gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vemetzungsagens.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Bevorzugt sind die kationischen Proteinhydrolysate und -derivate auf pflanzlicher Basis.

Weitere erfindungsgemäß einsetzbare pflegende Polymere sind amphotere Polymere.

Als Pflegestoff kann weiterhin mindestens ein Vitamin, ein Provitamin, eine Vitaminvorstufe und/oder eines derer Derivate eingesetzt werden.

Dabei sind erfindungsgemäß solche Vitamine, Pro-Vitamine und Vitaminvorstufen bevorzugt, die üblicherweise den Gruppen A, B, C, E, F und H zugeordnet werden. Besonders bevorzugt sind Vitamine, die zur B-Gruppe oder zu dem Vitamin B-Komplex gehören, ganz besonders bevorzugt Vitamin B₅ (Pantothensäure, Panthenol und Pantolacton).

Als Pflegestoff kann weiterhin mindestens ein Pflanzenextrakt eingesetzt werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Seerose, Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Als Pflegestoff eignen sich weiterhin eine Reihe von Carbonsäuren.

Vorteilhaft im Sinne der Erfindung können insbesondere kurzkettige Carbonsäuren sein. Unter kurzkettigen Carbonsäuren und deren Derivaten im Sinne der Erfindung werden Carbonsäuren verstanden, welche gesättigt oder ungesättigt und/oder geradkettig oder verzweigt oder cyclisch und/oder aromatisch und/oder heterocyclisch sein können und ein Molekulargewicht kleiner 750 aufweisen. Bevorzugt im Sinne der Erfindung können gesättigte oder ungesättigte geradkettigte oder verzweigte Carbonsäuren mit einer Kettenlänge von 1 bis zu 16 C-Atomen in der Kette sein, ganz besonders bevorzugt sind solche mit einer Kettenlänge von 1 bis zu 12 C - Atomen in der Kette.

Weitere geeignete Pflegestoffe sind Proteinhydrolysate und/oder deren Derivate, wobei die Verwendung von Proteinhydrolysaten pflanzlichen Ursprungs, z. B. Soja-, Mandel-, Erbsen-, Kartoffel- und Weizenproteinhydrolysaten, bevorzugt ist. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda), Crosilk^{®} (Croda) oder Crotein^{®} (Croda) vertrieben.

Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Weiterhin sind als Pflegestoff Lipide und Ölkörper, beispielsweise pflanzliche Öle, flüssige Paraffinöle, Isoparaffinöle, synthetische Kohlenwasserstoffe und Esteröle, Enzyme und Perlenextrakte geeignet.

Neben den Pflegestoffen können auch weitere Hilfs- und Zusatzstoffe zugegeben werden.

Durch Zugabe eines UV-Filters können sowohl die Zubereitungen selbst, als auch die behandelten Fasern vor schädlichen Einflüssen von UV-Strahlung geschützt werden. Es kann daher vorteilhaft sein, den pulverförmigen Zubereitungen mindestens einen UV-Filter zuzugeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Beispielhaft sei hier 2-Hydroxy-4-methoxybenzophenon-5-sulfonsäure und deren Natriumsalz (Benzophenone-4; Uvinul^{®}MS 40; Uvasorb^{®}S 5) genannt.

In einer besonderen Ausführungsform enthält die eingesetzte pulverförmige Zusammensetzung weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Auch die Zugabe eines Tensids ist nicht ausgeschlossen, jedoch wegen der bereits erwähnten nachteiligen Beeinflussung der Hydrophobizität des Siliciumdioxids und damit der Stabilität der pulverförmigen Zusammensetzung nicht bevorzugt.

Insbesondere zur Erhöhung der Stabilität der pulverförmigen Zusammensetzung kann es hingegen vorteilhaft sein, Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide, wie z.B. Polyvinylalkohol, zuzugeben.

Auch die übliche Zugabe von Parfümkomponenten und Konservierungsmitteln ist möglich.

Weiterhin können die pulverförmigen Zusammensetzungen Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Die pulverförmigen Zusammensetzungen, die erfindungsgemäß zur temporären Verformung keratinischer Fasern eingesetzt werden, lassen sich einfach herstellen. Es hat sich bewährt, zunächst auf bekannte Weise, etwa durch einfaches Verrühren, eine wässrige Lösung oder Dispersion des filmbildenden und/oder festigenden Polymers und der gewünschten Hilfs- und Zusatzstoffe herzustellen, diese in einem Mischer vorzulegen und schließlich das hydrophobierte Siliciumdioxidpulver unter intensivem Rühren zuzugeben. Die benötigte Mischzeit ist abhängig von der eingebrachten Mischenergie und der jeweiligen Zusammensetzung der Mischung, beträgt aber in der Regel zwischen 15 Sekunden und 5 Minuten. Wird zu kurz gemischt, bildet sich kein stabiles Pulver aus und es kommt zur Ausbildung einer wässrigen Phase. Bei zu langer Mischzeit wird das zunächst entstehende Pulver in eine brei- oder cremeartige Konsistenz überführt, wobei dieser Vorgang nicht reversibel verläuft. Es empfiehlt sich daher, durch einige Vorversuche die optimale Mischzeit für das jeweilige System zu ermitteln.

Die pulverförmigen Zusammensetzungen können in nahezu beliebigen Behältnissen konfektioniert werden. Es muss lediglich sichergestellt werden, dass die mechanische Belastung des Pulvers bei der Entnahme der Zusammensetzung nicht so hoch ist, dass bereits bei der Entnahme das Pulver in flüssige Form überführt wird. Geeignet sind beispielweise Tiegel, Flaschen oder auch Tetrapacks, wobei das Behältnis beispielsweise mit einer Schütt- und Dosiervorrichtung ausgestaltet werden kann. Bei der Verwendung der pulverförmigen Zusammensetzung zur temporären Verformung keratinischer Fasern wird zunächst die gewünschte Menge der pulverförmigen Zusammensetzung dem Behältnis entnommen. Die Zusammensetzung kann dabei direkt auf die zu behandelnde keratinische Faser oder aber beispielsweise auf die Hand gegeben werden. Im ersten Fall kann das aufgebrachte Pulver direkt auf der keratinischen Faser einer mechanischen Belastung, beispielsweise mittels der Hände ausgesetzt werden, wodurch das Lösungsmittel und das filmbildende und/oder festigende Polymer direkt auf der Faser freigesetzt werden. Wird die pulverförmige Zusammensetzung zunächst auf die Hand gegeben, so kann es zunächst vorsichtig im Haar verteilt und wiederum erst anschließend stärk mechanisch belastet werden, beispielsweise durch gezieltes Einmassieren des Pulvers in das Haar. Dadurch wird das haltgebende Polymer erst direkt auf der gewünschten Haarpartie freigesetzt. So lässt sich sehr gezielt eine hervorragende Stylingwirkung erreichen. Es ist natürlich auch möglich, die pulverförmige Zusammensetzung bereits auf der Hand zu verreiben und erst das entstehende flüssige oder pastenartige Mittel auf die keratinische Faser aufzubringen. Diese Vorgehensweise ist allerdings nicht bevorzugt, da dabei auf einen wesentlichen Vorteil der pulverförmigen Konsistenz des Stylingmittels, nämlich die gute Verteilbarkeit, verzichtet wird. Die pulverförmige Zusammensetzung lässt sich natürlich auch mit einem Hilfsmittel, etwa einem Pinsel, einem Schwamm, einem Tuch, einer Bürste oder einem Kamm auftragen.

Ein zweiter Gegenstand der Erfindung sind pulverförmige Stylingmittel, enthaltend 50 bis 90 Gew.-% eines wässrigen Lösungsmittels, hydrophobiertes Siliciumdioxidpulver und 5,5 bis 15 Gew.-% mindestens eines filmbildenden und/oder festigenden Polymers.

Die besonderen und bevorzugten Ausgestaltungen des erfindungsgemäßen pulverförmigen Stylingmittels entsprechen dem bereits oben Ausgeführten.

Die nachfolgenden Beispiele sollen den Gegenstand der vorliegenden Erfindung erläutern ohne ihn in irgendeiner Weise zu beschränken.

### Beispiele:

Die im folgenden angegebenen Mengenangaben verstehen sich - soweit nichts anderes vermerkt ist - in Gewichtsprozent.

### 1 Herstellung flüssiger Stylingmittel

Es wurden zunächst auf übliche Weise die flüssigen Stylingmittel A1 bis A4 hergestellt, wobei diese folgende Zusammensetzung aufwiesen:

| **Rohstoffe** | **A1** | **A2** | **A3** | **A4** |
|---|---|---|---|---|
| Luviskol^{®} VA 37 E 60 | 8,0 | - | - | 6,0 |
| PVP/VA 60/40 W NP | - | 4,0 | - | - |
| Aristoflex^{®} A 60 | - | 5,0 | - | - |
| Advantage^{®} LC-E | - | - | 10,0 | - |
| Gafquat^{®} 755N | - | - | - | 0,5 |
| Genamin^{®} CTAC | 0,3 | 0,1 | 0,2 | 0,15 |
| Gluadin^{®} WQ | - | - | 0,25 | - |
| Gluadin^{®} WLM | - | - | 0,25 | - |
| Dow Corning^{®} 193 Surfactant | - | 0,2 | - | - |
| PEG-40 Hydrogenated Castor Oil | - | 0,1 | - | 0,1 |
| Polyethylenglykol (MG 1500) | - | 0,2 | - | 0,6 |
| Herb. Extrakt Seerose COS-241/404-A | - | - | 0,1 | - |
| Solan^{®} ELD | - | - | 0,2 | - |
| D-Panthenol (75 %) | - | - | 0,2 | - |
| Pantolacton | - | - | 0,2 | - |
| Benzophenone-4 | - | - | 0,05 | - |
| Glycin | - | - | 0,2 | - |
| Milchsäure (80 %) | - | - | 0,05 | - |
| Parfüm | 0,1 | 0,1 | 0,2 | 0,1 |
| Ethanol (96 %), vergällt | 32,0 | 52,0 | 30,0 | 30,0 |
| Wasser, entsalzt | ad 100 | ad 100 | ad 100 | ad 100 |

### 2 Überführung in erfindungsgemäße pulverförmige Zusammensetzungen

47,5 g des jeweiligen flüssigen Stylingmittels A1, A2, A3 oder A4 wurden in einem Mischer vorgelegt und unter Rühren mit jeweils 2,5 g des hydrophobierten Siliciumdioxidpulvers Aerosil^{®} R 812 S (INCl-Bezeichnung: Silica Silylate) versetzt. Nach einer Rührzeit von jeweils 30 bis 45 Sekunden hatte sich jeweils ein stabiles Pulver gebildet, das in Polyethylenflaschen abgefüllt wurde. Auf diese Weise resultierten die pulverförmige Zusammensetzungen B1, B2, B3 und B4.

### 3 Anwendung

Zur Anwendung wurde jeweils die gewünschte Menge der pulverförmigen Zusammensetzungen B1, B2, B3 bzw. B4 entnommen und vorsichtig in Humanhaar verteilt. Anschließend wurde die Zusammensetzung durch Massieren und Kneten verflüssigt und dabei das Haar mit den Händen in die gewünschte Form gebracht. Es wurde ein ausgezeichnetes Stylingergebnis erhalten, wobei überraschenderweise weder Pulverrückstände im behandelten Haar sichtbar waren, noch ein übermäßiger Mattierungseffekt festgestellt wurde.

### 4 Verzeichnis der eingesetzten Rohstoffe

Die im Rahmen der Beispiele eingesetzten Rohstoffe sind wie folgt definiert:
- Luviskol^{®} VA 37 E60: Vinylpyrrolidon Vinylacetat Copolymer (30:70) (ca. 48-52% Festkörper in Ethanol; INCI-Bezeichnung: VPNA Copolymer) (BASF)
- PVPNA 60/40 W NP: Vinylpyrrolidon Vinylacetat Copolymer (60:40) (ca. 48-52% Festkörper in Wasser; INCI-Bezeichnung: VPNA Copolymer) (ISP)
- Aristoflex^{®} A 60: Vinylacetat Crotonsäure Copolymer (ca. 60-63% Festkörper in i-Propylalkohol; INCI-Bezeichnung: VA/Crotonates Copolymer, Isopropyl Alcohol) (Clariant)
- Advantage^{®} LC-E: Vinylcaprolactam Vinylpyrrolidon Dimethylaminoethylmethacrylat Copolymer (ca. 35-39% Festkörper in Ethanol; INCI-Bezeichnung: Vinyl Caprolactam/VP/Dimethylaminoethyl Methacrylate Copolymer, Alcohol, Lauryl Pyrrolidone) (ISP)
- Gafquat^{®} 755 N: Dimethylaminoethylmethacrylat Vinylpyrrolidon Copolymer, quaterniert mit Diethylsulfat (ca. 19% Festkörper in Wasser; INCI-Bezeichnung: Polyquaternium-11) (ISP)
- Genamin^{®} CTAC: Trimethylhexadecylammoniumchlorid (ca. 28-30% Aktivsubstanz in Wasser; INCI-Bezeichnung: Cetrimonium Chloride) (Clariant)
- Gluadin^{®} WQ: Weizenproteinhydrolysat (ca. 31-35% Festkörper; INCI-Bezeichnung: Aqua (Water), Laurdimonium Hydroxypropyl Hydrolyzed Wheat Protein, Ethylparaben, Methylparaben) (Cognis)
- Gluadin^{®} WLM: Weizenproteinhydrolysat (ca. 21-24% Festkörper; INCI-Bezeichnung: Hydrolyzed Wheat Protein) (Cognis)
- Dow Corning^{®} 193 surfactant: Silikon Glykol Copolymer (INCI-Bezeichnung: PEG-12 Dimethicone) (Dow Corning)
- PEG-40 Hydrogenated Castor Oil:: Polyethylenglykol-Derivat des hydrierten Rizinusöls mit durchschnittlich 40 Mol Ethylenoxid (INCI-Bezeichnung: PEG-40 Hydrogenated Castor Oil) (BASF)
- Solan^{®} ELD:: Polyethylenglykol-Derivat des Lanolins mit durchschnittlich 75 Mol Ethylenoxid (INCI-Bezeichnung: PEG-75 Lanolin) (Croda)
- Benzophenone-4 (INCl): 2-Hydroxy-4-methoxybenzophenon-5-sulfonsäure

## Patentansprüche

1. Verwendung einer pulverförmigen Zusammensetzung, enthaltend
- 50 bis 96 Gew.-% eines wässrigen Lösungsmittels,
- hydrophobiertes Sillciumdloxidpulver und
- mindestens ein filmbildendes und/oder festigendes Polymer, wobei unter filmbildenden Polymeren solche Polymere verstanden werden, die bei Anwendung in 0,01 bis 20 Gew.-%-iger wässriger, alkoholischer oder wässrigalkoholischer Lösung auf dem Haar einen transparenten Polymerfilm abscheiden,
zur temporären Verformung keratinischer Fasern.

2. Verwendung gemäß Anspruch 1, **dadurch gekennzeichnet, dass** die pulverförmige Zusammensetzung 70 bis 90 Gew.% eines wässrigen Lösungsmittels enthält.

3. Verwendung gemäß wenigstens eines der Ansprüche 1 und 2, **dadurch gekennzeichnet, dass** es sich bei dem wässrigen Lösungsmittel um Wasser oder ein Wasser/Ethanol-Gemisch handelt.

4. Verwendung gemäß wenigstens eines der Ansprüche 1 bis 3, **dadurch gekennzeichnet, dass** die pulverförmige Zusammensetzung 0,5 bis 15 Gew.% eines hydrophobierten Siliciumdioxidpulvers enthält.

5. Verwendung gemäß wenigstens eines der Ansprüche 1 bis 4, **dadurch gekennzeichnet, dass** die pulverförmige Zusammensetzung 5,5 bis 15 Gew.-% mindestens eines filmbildenden und/oder festigenden Polymers enthält.

6. Verwendung gemäß wenigstens eines der Ansprüche 1 bis 5, **dadurch gekennzeichnet, dass** die pulverförmige Zusammensetzung mindestens ein filmbildendes und/oder festigendes Polymer enthält, das aus mindestens einem der folgenden Monomere aufgebaut ist: Vinylpyrrolidon, Vinylcaprolactam, Vinylester, Vinylalkohol, Acrylamid, Methacrylamid, C₁- bis C₇-Alkyl- und C₁- bis C₇-Dialkylacrylamid, C₁- bis C₁-Alkyl- und C₁- bis C₇-Dialkylmethacrylamid, C₁- bis C₇-Alkylacrylat, C₁- bis C₇-Alkylmethacrylat, Propylenglykol und Ethylenglykol.

7. Verwendung gemäß Anspruch 6, **dadurch gekennzeichnet, dass** die filmbildenden und/oder festigenden Polymere ausgewählt sind aus Vinylpyrrolidon-Vinylacetat-Copolymeren, Vinylacetat-Crotonsäure-Copolymeren, Vinylcaprolactam-Vinylpyrrolidon-DimethylaminoethylmethacrylatCopolymeren, Octylacrylamid-Acrylat-Butylaminoethyl-Methacrylat-Copolymeren und quaternierten Vinylpyrrolidon-Dimethylaminoethylmethacrylat-Copolymeren.

## Claims

1. Use of a pulverulent composition, containing
- 50 to 95 wt.% of an aqueous solvent,
- hydrophobised silicon dioxide powder and
- at least one film-forming and/or setting polymer, film-forming polymers being taken to mean those polymers which, when used in a 0.01 to 20 wt.% aqueous, alcoholic or aqueous-alcoholic solution on the hair, deposit a transparent polymer film,
for temporary deformation of keratinic fibres.

2. Use according to claim 1, **characterised in that** the pulverulent composition contains 70 to 90 wt.% of an aqueous solvent.

3. Use according to at least one of claims 1 and 2, **characterised in that** the aqueous solvent is water or a water/ethanol mixture.

4. Use according to at least one of claims 1 to 3, **characterised in that** the pulverulent composition contains 0.5 to 15 wt.% of a hydrophobised silicon dioxide powder.

5. Use according to at least one of claims 1 to 4, **characterised in that** the pulverulent composition contains 5.5 to 15 wt.% of at least one film-forming and/or setting polymer.

6. Use according to at least one of claims 1 to 5, **characterised in that** the pulverulent composition contains at least one film-forming and/or setting polymer synthesised from at least one of the following monomers: vinylpyrrolidone, vinyl caprolactam, vinyl ester, vinyl alcohol, acrylamide, methacrylamide, C₁ to C₇ alkyl- and C₁ to C₇ dialkylacrylamide, C₁ to C₇ alkyl- and C₁ to C₇ dialkylmethacrylamide, C₁ to C₇ alkyl acrylate, C₁ to C₇ alkyl methacrylate, propylene glycol and ethylene glycol.

7. Use according to claim 6, **characterised in that** the film-forming and/or setting polymers are selected from vinylpyrrolidone-vinyl acetate copolymers, vinyl acetate-crotonic acid copolymers, vinyl caprolactamvinylpyrrolidone-dimethylaminoethyl methacrylate copolymers, octylacrylamide-acrylate-butylaminoethyl-methacrylate copolymers and quaternised vinylpyrrolidone-dimethylaminoethyl methacrylate copolymers.

## Revendications

1. Utilisation d'une composition pulvérulente contenant :
- de 50 à 95 % en poids d'un solvant aqueux ;
- de la poudre de dioxyde de silicium rendu hydrophobe ; et
- au moins un polymère filmogène et/ou renforçateur, dans laquelle, par l'expression « polymères filmogènes », on entend des polymères qui, lorsqu'on les utilise dans 0,01 à 20 % en poids d'une solution aqueuse, alcoolique ou aqueuse-alcoolique, déposent sur les cheveux un film polymère transparent,
pour la déformation temporaire de fibres kératiniques.

2. Utilisation selon la revendication 1, **caractérisée en ce que** la composition pulvérulente contient de 70 à 90 % en poids d'un solvant aqueux.

3. Utilisation selon au moins une des revendications 1 et 2, **caractérisée en ce que**, en ce qui concerne le solvant aqueux, il s'agit d'eau ou d'un mélange eau/éthanol.

4. Utilisation selon au moins une des revendications 1 à 3, **caractérisée en ce que** la composition pulvérulente contient de 0,5 à 15 % en poids d'une poudre de dioxyde de silicium rendu hydrophobe.

5. Utilisation selon au moins une des revendications 1 à 4, **caractérisée en ce que** la composition pulvérulente contient de 5,5 à 15 % en poids d'au moins un polymère filmogène et/ou renforçateur.

6. Utilisation selon au moins une des revendications 1 à 5, **caractérisée en ce que** la composition pulvérulente contient au moins un polymère filmogène et/ou renforçateur qui est constitué par au moins un des monomères suivants : la vinylpyrrolidone, le vinylcaprolactame, un ester vinylique, l'alcool vinylique, l'acrylamide, le méthacrylamide, des alkyl(en C₁-C₇)- et dialkyl(en C₁-C₇)-acrylamides, des alkyl(en C₁-C₇)- et dialkyl(en C₁-C₇)-méthacrylamides, des acrylates d'alkyle en C₁-C₇, des méthacrylates d'alkyle en C₁-C₇, le propylèneglycol et l'éthylèneglycol.

7. Utilisation selon la revendication 6, **caractérisée en ce que** les polymères filmogènes et/ou renforçateurs sont choisis parmi des copolymères de vinylpyrrolidone-acétate de vinyle, des copolymères d'acétate de vinyle-acide crotonique, des copolymères de vinylcaprolactamevinylpyrrolidone-méthacrylate de diméthylaminoéthyle, des copolymères d'octylacrylamide-acrylate-méthacrylate de butylaminoéthyle et des copolymères quaternisés de vinylpyrrolidone-méthacrylate de diméthylaminoéthyle.
